# EUROPÄISCHE PATENTSCHRIFT

(11) **EP 2 082 725 B1**
(45) Veröffentlichungstag und Bekanntmachung des Hinweises auf die Patenterteilung: **12.10.2016**
(21) Anmeldenummer: 09150407.6
(22) Anmeldetag: 12.01.2009
(51) Int. Cl.: A61K 8/49, A61K 8/97, A61Q 17/04, A61Q 19/00

(54) **Sonnenschutz-Kosmetikset und kosmetisches Verfahren zur Behandlung sensitiver Haut**
Sunscreen cosmetic set and cosmetic method for treating sensitive skin
Ensemble cosmétique de protection solaire et procédé cosmétique destiné au traitement de la peau sensible

(30) Priorität: 11.01.2008 DE 102008004664
(43) Veröffentlichungstag der Anmeldung: 29.07.2009
(73) Patentinhaber: Coty Germany GmbH, 55116 Mainz (DE)
(72) Erfinder: Golz-Berner, Karin, 98000 Monaco (MC); Zastrow, Leonhard, 98000 Monaco (MC)
(74) Vertreter: Gulde & Partner

(56) Entgegenhaltungen:
- WO-A1-2009/044040
- DE-B3-102004 020 060
- FR-A1- 2 899 106
- JP-A- 2002 128 630
- SRIVASTAVA RITU ET AL: "Chemistry, pharmacology and botany of Boerhaavia diffusa - a review", CURRENT RESEARCH ON MEDICINAL AND AROMATIC PLANTS, CENTRAL INSTITUTE OF MEDICINAL AND AROMATIC PLANTS, LUCKNOW, IN, Bd. 20, Nr. 3, 1. Januar 1998 (1998-01-01) , Seiten 762-767, XP009101214, ISSN: 0253-7125

## Beschreibung

Die Erfindung betrifft ein Sonnenschutz-Kosmetikset und ein kosmetisches Verfahren zur Behandlung sensitiver Haut.

Bei der Anwendung kosmetischer Zusammensetzungen kommt es in nicht wenigen Fällen zu Irritationen der Haut, da ein Teil der Anwenderinnen und Anwender empfindlich auf bestimmte Inhaltsstoffe reagieren. Zu solchen Substanzen, die zu Hautirritationen bei diesen Anwendern führen können, gehören beispielsweise die meisten organischen Filtersubstanzen für UVA- oder UVB-Strahlung, einige Konservierungsmittel sowie überraschenderweise auch eine Reihe von synthetischen Siliconölen. Für eine empfindliche Haut ist daher das Weglassen oder wenigstens die Reduzierung derartiger Stoffe mit irritativer Wirkung angezeigt. Dies ist jedoch aus Gründen des notwendigen Hautkomforts, der notwendigen Lagerstabilität und des Schutzes gegen freie Radikale nur schwierig in einer kosmetischen Zusammensetzung zu vereinen.

Es besteht daher die Aufgabe, mit einer kosmetischen Zusammensetzung einen ausreichenden Sonnenschutz für sensitive Haut bereitzustellen und dabei gleichzeitig Hautirritationen weitgehend zu vermeiden.

Eine weitere Aufgabe besteht in der Bereitstellung eines kosmetischen Verfahrens zur Behandlung sensitiver Haut.

Erfindungsgemäß wird die Aufgabe mit einem Sonnenschutz-Kosmetikset für empfindliche Haut gelöst, das (a) eine Sonnenschutzzubereitung für den Bereich SPF 8 bis SPF 20 mit einem Gemisch von UVA- und UVB-Filtern und einem Gehalt an Siliconöl(en), der im Bereich von 1,2 bis 3,5 Gew.-% liegt, (b) eine Serumzubereitung ohne Siliconöl und (c) eine Cremezubereitung ohne Siliconöl umfasst, wobei die Zubereitungen jeweils ein Wirkstoffgemisch enthalten, bestehend aus Anthemis Nobilis Flower Extract, Hesperetin Laurate und Boerhavia Root Extract, sowie Calendula officinalis flower extract und der Gehalt an Konservierungsmitteln in allen Zubereitungen jeweils im Bereich von 0,01 bis 0,35 Gew.-% liegt bei einer Lagerstabilität von wenigstens 24 Monaten für alle Zubereitungen, und
wobei das Verhältnis der Anteile des Wirkstoffgemisches in der Serumzubereitung zu den Anteilen des Wirkstoffgemisches in der Sonnenschutzzubereitung und in der Cremezubereitung im Bereich von 1 : 0,3 - 0,8 : 0,3 - 0,8 liegt.

Die Lagerstabilität von wenigstens 24 Monaten wurde bei einer Lagerung bei Raumtemperatur bei 20 bis 25 °C und 55-60 % Luftfeuchtigkeit erreicht.

Der Anteil der einzelnen Wirkstoffe liegt vorteilhaft im Bereich von 0,3 bis 2,2 Gew.-%, vorzugsweise im Bereich von 0,3 bis 2,0 Gew.-% bezogen auf das Gesamtgewicht der Einzelzubereitungen, d.h. der Serumzubereitung bzw. der Sonnenschutzzubereitung bzw. der Cremezubereitung. Die Anteile der einzelnen Wirkstoffe im Wirkstoffgemisch können gleich oder unterschiedlich groß sein. Vorteilhafterweise liegt der Anteil von Hesperitin Laurate im Bereich von 0,3 bis 2,0 Gew. % bezogen auf das Gesamtgewicht der Einzelzubereitungen.

Der Anteil des Wirkstoffgemisches liegt vorteilhaft im Bereich von 0,9 bis 6,6 Gew.-%, vorzugsweise im Bereich von 0,9 bis 4 Gew.-% bezogen auf das Gesamtgewicht der Einzelzubereitungen.

Die einzelnen Bestandteile des Kosmetiksets der Erfindung werden nacheinander zu unterschiedlichen Zeiten verwendet. Dies ist Gegenstand des erfindungsgemäßen Verfahrens.

Die Sonnenschutzzubereitung (a) ist durch ihre UV-strahlungsaktiven Filtersubstanzen so eingestellt, daß sie einen Bereich des Sonnenschutzfaktors (Sun Protecting Factor) SPF von 8 bis 20 abdeckt. Sonnenschutzzubereitungen mit einem SPF > 20 enthalten hohe Mengen an organischen Filtersubstanzen und sind daher für eine sensitive Haut in den meisten Fällen nicht geeignet.

Als Filtersubstanzen werden übliche wasser- und/oder öllösliche UVB-Filter und UVA-Filter eingesetzt.

Für die Erfindung ist wesentlich, dass in jedem Falle ein Gemisch von UVA- und UVB-Filtern eingesetzt wird, bei dem der UVA-Filteranteil vorteilhaft im Bereich von 20-40 Gew-% liegt, vorzugsweise 30-40 Gew-%, bezogen auf das Gesamtgemisch UVA- zu UVB-Filter.

Besonders bevorzugte Filter sind Benzophenone-3, Butyl-Methoxybenzoylmethane, Octyl Methoxycinnamate, Octyl Salicylate, 4-Methylbenzylidene Camphor, Homosalate und Octyl Dimethyl PABA.

Bevorzugte wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion, Butyl Methoxybenzoyl-methane oder Menthyl Anthranilate.

Besonders bevorzugt sind Benzophenone-3, Butyl Methoxydibenzoylmethane, Octyl Methoxycinnamate, Octyl Salicylate, 4-Methylbenzylidene Camphor, Homosalate, Octocrylene, Ethylhexyl Methoxycinnamate, Isoamyl-p-Methoxycinnamate, Octyl Dimethyl PABA, Ethylhexyltriazone, Diethylhexyl Butamido Triazone, Ethylhexyl Salicylate, Methylene Bis-Benzotriazolyl Tetramethylbutylphenol, Disodium Phenyl Dibenzimidazole Tetrasulfonate, Bis-Ethylhexyloxyphenol Methoxyphenyl Triazine und Gemische davon.

Weiterhin können auch Breitbandfilter eingesetzt werden, wie z.B. Bis-Resorcinyltriazin-Derivate, oder auch Benzoxazole oder UV-Pearls® (Merck, Deutschland), letztere bestehend aus Wasser, Ethylhexyl Methoxycinnamate, Silica, PVP, Ethanol, Natriumcitrat, Chlorphenesin, Centrimonium Chloride.

Die Bestimmung des Sonnenschutzfaktors SPF erfolgt üblicherweise nach dem COLIPA-Verfahren (Colipa-Ref.: 94/289 (1994)), wobei im wesentlichen nur UVB-Strahlung erfasst wird. Die *in vivo*-UVA-Schutzfaktoren werden nach dem Persistant Pigment Darkening-Verfahren (PPD) als Endpunkt (Photodermatol. Photoimmunol. Photomed. 16, 245-249 (2000)) ermittelt.

Der Sonnenschutzzubereitung zugesetzt wird ein Wirkstoffgemisch aus Anthemis Nobilis Flower Extract, beispielsweise erhältlich als Chamomilla Ecoconcentrate Natural (Robertet S.A.), Hesperetin Laurate, beispielsweise erhältlich als Flavagrum® PEG (Coletica, Frankreich) und Boerhavia Root Extract, beispielsweise erhältlich als Mediacalm® (Silab, sowie Calendula officinalis flower extract. Das Gemisch aus zwei Pflanzenextrakten und einem Fettsäureester mit Flavon-ähnlicher Struktur zeigt eine überraschend gute Verträglichkeit mit sensitiver Haut und führt in einer Kombination mit UVA- und UVB-Filtern kaum zu Hautirritationen, sofern die Menge der Filter die zur Erreichung des SPF 20 erforderliche Filtermenge nicht überschreitet.

Weiterhin überraschend ist die Tatsache, daß ein bestimmter Konservierungseffekt auftritt, der eine Begrenzung von zugesetzten Konservierungsmitteln auf maximal 0,35 Gew-%, vorzugsweise maximal 0,3 Gew.-%, ermöglicht jeweils bezogen auf die Sonnenschutzzubereitung, die Serumzubereitung bzw. die Cremezubereitung. Dies ist vorteilhaft, da Konservierungsmittel geeignet sind, Irritationen der Haut, insbesondere von sensitiver Haut hervorzurufen. Der Konservierungseffekt kann insbesondere durch den Zusatz eines Boosters, vorzugsweise in Form eines Gemisches aus 1,2-Hexandiol, 1,2-Octanediol und Tropolone (INCI: 1,2-Hexandiol & Caprylyl Glycol & Tropolone), beispielsweise erhältlich als Symdiol® 68T von Symrise verbessert werden.

Das Wirkstoffgemisch enthält neben den eigentlichen Wirkstoffen noch Hilfsstoffe wie Wasser, mehrwertige Alkohole wie Propylenglycol, Butylenglycol oder Ester wie PEG-6 Isostearate.

Den kosmetischen Zubereitungen (a), (b) und (c) wird zusätzlich zu dem eigentlichen Wirkstoffgemisch Calendula officinalis flower extract beispielsweise erhältlich als Calendulaöl (Provital oder CLR) zugesetzt. Vorzugsweise liegt der Gehalt an Calendulaöl im Bereich von 0,1 bis 2 Gew.-%, vorzugsweise im Bereich von 0,5 bis 1,5 Gew.-%, noch bevorzugter bei 1,0 oder 1,2 Gew.-%.

Die Sonnenschutzzubereitung kann weitere Hilfsstoffe enthalten, wie Radikalfänger, Wasser, mehrwertige Alkohole, Gelbildner, Allantoin, Vitamine, Calendulaöl, Shea Butter, Chelatisierungsmittel, Ester und/oder Ether zur Bildung einer Ölphase und gegebenenfalls weitere Hilfsstoffe.

Die Sonnenschutzzubereitung enthält nur eine verhältnismäßig geringe Menge an Siliconölen, wie sie zur Lösung der Filtersubstanzen erforderlich ist, und verzichtet darüber hinaus auf synthetische Siliconöle.

Die erfindungsgemäße Serumzubereitung umfasst übliche Serumbestandteile wie Wasser, mehrwertige Alkohole, Copolymere, Chelatisierungsmittel, Fettsäureester, Isohexadecan, Shea Butter, Calendulaöl, Radikalfänger und Bisabolol sowie das obengenannte Wirkstoffgemisch, Konservierungsstoffe und gegebenenfalls weitere Hilfsstoffe.

Die Serumzubereitung enthält keine Öle, insbesondere keine Siliconöle.

Die erfindungsgemäße Cremezubereitung umfasst übliche Cremebestandteile wie Wasser, mehrwertige Alkohole, Gelbildner, Allantoin, Vitamine, Radikalfänger, Ester und/oder Ether zur Bildung einer Ölphase, Shea Butter, Allantoin, Calendulaöl, Bisabolol (z.B. Dragosantol von Symrise) und Copolymere wie Aristoflex® (Clariant) und gegebenenfalls weitere Hilfsstoffe sowie das oben genannte Wirkstoffgemisch und Konservierungsmittel.

Die Cremezubereitung enthält keine Öle, insbesondere keine Siliconöle.

Als Antioxidationsmittel oder Radikalfänger können eingesetzt werden Vitamine wie Vitamin C und Derivate davon, beispielsweise Ascorbylacetat, -phosphat und - palmitat, Magnesiumascorbylphosphat; Folsäure und deren Derivate; Vitamin E und deren Derivate, wie Tocopherylacetat; Flavone oder Flavonoide; Aminosäuren, wie Histidin, Glycin, Tyrosin, Tryptophan und Derivate davon; Imidazole wie z.B. cis- oder trans-Urocaninsäure und deren Derivate; Peptide wie D,L-Carnosin, D-Carnosin, L-Carnosin und deren Derivate; Carotinoide und Carotine, wie z.B α-Carotin, β-Carotin, Lycopin; Harnsäure und Derivate davon; α-Hydroxyfettsäuren wie Palmitinsäure, Phytinsäure, Lactoferrin; Stilbene und deren Derivate; Mannose und deren Derivate; Ferulasäure und deren Derivate; Thiole wie Glutathion, Cystein, Cystin und deren Ester.

Ein besonders bevorzugter Radikalfänger ist ein Gemisch mit einem Gehalt an einem durch Extraktion der Rinde von Quebracho blanco und nachfolgender enzymatischer Hydrolyse gewonnenem Produkt, das wenigstens 90 Gew- % Proanthocyanidin-Oligomere und höchstens 10 Gew-% Gallussäure enthält, in Mikrokapseln, sowie mit einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser (RPF-Komplex 1).

In einer bevorzugten Ausführungsform enthalten die Sonnenschutzzubereitung, die Serumzubereitung und die Cremezubereitung jeweils einen Radikalfänger oder ein Gemisch von Radikalfängern. Vorzugsweise werden der Radikalfänger oder das Gemisch an Radikalfängern ausgewählt aus der Gruppe bestehend aus Vitaminen, Vitaminderivaten, Flavonen, Flavonoiden, Enzymen, einem als RPF-Komplex 1 bezeichneten Gemisch von Quebracho blanco-Extrakt, Seidenraupenextrakt, Hydro-Gel, Phospholipiden und Wasser oder einer Mischung davon.

Ein weiteres bevorzugtes Antioxidationsmittel ist ein Gemisch aus Angelica archangelica-Wurzelextrakt, Pongamia pinnata-Samenextrakt, Camelia sinensis-Blattextrakt und Coffea arabica-Samenextrakt. Vorzugsweise wird ein RPF-Komplex (als "RPF-Komplex classic" bezeichnet) eingesetzt, der ein in Lecithin verkapseltes Gemisch aus (in Gew.-%) 2 % Angelica archangelica-Wurzelextrakt (INCI: Angelica archangelica root extract, CAS number 84775-41-7), 2 % Pongamia pinnata-Samenextrakt (INCI: Pongamia pinnata seed extract), 2 % Camelia sinensis-Blattextrakt (INCI: Camelia sinensis Leaf Extract, CAS number 84650-60-2), 2 % Coffea arabica-Samenextrakt (INCI: Coffea arabica (coffee) seed extract, CAS number 84650-00-0) beinhaltet, wobei außerdem 8 % Glycerin, 8,25 % Alkohol denaturiert, Antioxidationsmittel und Hilfsstoffe enthalten sind.

Als Ester oder Ether zur Bildung der Ölphase können beispielsweise eingesetzt werden Dipentaerythrityl Hexacaprilate/Hexacaprate, Tridecyl Trimellitate/Tridecyl Stearate, Neopentyl Glycol Dicaprylate, Propylene Glycol Dioctanoate, Propylene Glycol Dicaprylate-2,30-Dicaprate, Tridecyl Stearate/Neopentyl Glycol Dicaprylate Dicaprate, Tridecyl Trimellitate, Neopentyl Glycol Dioctanoate, Isopropyl Myristate, Diisopropyl Dimer Dilinoleate, Trimethylpropane Triisostearate, Myristyl Ether, Stearyl Ether, Cetearyl Octanoate, Butyl Ether, Dicaprylyl Ether, PPG1-PEG9 Lauroyl Glycol Ether, PPG15 Stearyl Ether, PPG14 Butyl Ether, Fomblin HC25, Steareth-21, Steareth-2, Sorbitan Oleate.

Vorzugsweise liegt das Verhältnis der Anteile des Wirkstoffgemisches in der Serumzubereitung zu den Anteilen des Wirkstoffgemisches in der Sonnenschutzzubereitung und in der Cremezubereitung im Bereich von 1 : 0,4 - 0,7 : 0,4 - 0,8. Es wurde gefunden, daß ein erhöhter Anteil Wirkstoffgemisch in der Serumzubereitung und im Zusammenhang mit dem Einsatz dieser Zubereitung in dem kosmetischen Verfahren zu ausgezeichneten Ergebnissen führt, bei denen keine Irritationswirkung bei sensitiver Haut in den entsprechenden Tests beobachtet werden konnte.

Erfindungsgemäß enthalten die einzelnen Zubereitungen des Kosmetiksets der Erfindung kein Parfümöl, wodurch für die sensitive Haut eine weitere Quelle für Irritationen vermieden wird. Zur geruchlichen Verbesserung können natürliche Aromen, wie Lavendel, Rose, Vanille oder Mischungen davon zugesetzt werden.

Das erfindungsgemäße kosmetische Verfahren zur Behandlung sensitiver Haut besteht darin, daß die Haut
(a) am Morgen mit einer Serumzubereitung behandelt wird, umfassend ein Wirkstoffgemisch, bestehend aus Anthemis Nobilis Flower Extract, Hesperetin Laurate und Boerhavia Root Extract, Konservierungsmittel im Bereich von 0,01 bis 0,35 Gew-% und kein Siliconöl; und
(b) nach einer Einwirkzeit des Serums (a) von 0,5 bis 60 Minuten, vorzugsweise 1 bis 20 Minuten, noch bevorzugter 5 bis 10 Minuten, mit einer Sonnenschutzzubereitung für den Bereich SPF 8 bis SPF 20 behandelt wird, umfassend ein Gemisch von UVA- und UVB-Filtern, ein oder mehrere Siliconöle mit einem Anteil im Bereich von 1,2 bis 3,5 Gew.-%, ein Wirkstoffgemisch, bestehend aus Anthemis Nobilis Flower Extract, Hesperetin Laurate und Boerhavia Root Extract, und Konservierungsmittel, wobei der Anteil der Konservierungsmittel im Bereich von 0,01 bis 0,35 Gew.-% liegt;
(c) am Abend, vorzugsweise nach 8 bis 14 Stunden, noch bevorzugter nach 10 bis 12 Stunden, mit einem Serum gemäß (a) behandelt wird; und
(d) nach einer Einwirkzeit von 0,5 bis 60 Minuten, vorzugsweise 1 bis 20 Minuten, noch bevorzugter 5 bis 10 Minuten, mit einer Cremezubereitung behandelt wird, umfassend ein Wirkstoffgemisch aus Anthemis Nobilis Flower Extract, Hesperetin Laurate und Boerhavia Root Extract, und Konservierungsmittel, wobei der Anteil der Konservierungsmittel im Bereich von 0,01 bis 0,35 Gew.-% liegt und kein Siliconöl enthalten ist. Dabei liegt das Verhältnis der Anteile des Wirkstoffgemisches in dem Serum zu den Anteilen des Wirkstoffgemisches in der Sonnenschutzzubereitung und in der Creme im Bereich von 1 : 0,3-0,8 : 0,3-0,8, vorzugsweise im Bereich von 1 : 0,3 - 0,7 : 0,3 - 0,7.

Alle Prozentangaben sind jeweils auf das Gesamtgewicht der Serumzubereitung oder der Sonnenschutzzubereitung oder der Cremezubereitung entsprechend bezogen.

Es wurde weiterhin gefunden, dass bei Einsatz der einzelnen Bestandteile des erfindungsgemäßen Kosmetiksets diese Einzelbestandteile (Sonnenschutz, Serum, Creme) jeweils eine Lagerstabilität von 36 Monaten bei Raumtemperatur bei 20 bis 25 °C und 55-60% Luftfeuchtigkeit hatten.

Die Erfindung soll nachstehend durch Beispiele näher erläutert werden. Alle Angaben erfolgen in Gewichtsprozenten, sofern nichts anderes angegeben ist.

### Beispiel 1 Sonnenschutzzubereitung I SPF 15

| **Phase A** | |
|---|---|
| Wasser | q.s. ad 100 |
| Glycerine | 2,0 |
| Carbomer | 0,6 |
| Allantoin | 0,05 |
| Bisabolol | 0,3 |

| **Phase B** | |
|---|---|
| Dimethicone | 3,0 |
| Shea Butter | 4,0 |
| Dicaprylyl Carbonate &Tocopherol | 4,0 |
| Butyl Methoxydibenzoyl Methane | 2,0 |
| Ethylhexyl Methoxycinnamate | 7,5 |
| Octocrylene | 2,0 |
| Steareth-21 | 2,0 |
| Steareth-2 | 4,0 |

| **Phase C** | |
|---|---|
| Triethanolamin | 0,5 |

| **Phase D** | |
|---|---|
| Anthemis Nobilis Flower Extract | 1,0 |
| Calendula Oil | 1,0 |
| Boerhavia Diffusa Root Extract & Butylene Glycol | 0,5 |
| Hesperetin Laurate & PEG-6 Isostearate | 0,5 |
| Konservierungsmittel | 0,3 |
| Booster | 0,5 |
| Aroma Lavendel | 0,3 |

Ein bevorzugtes Carbomer ist beispielsweise Acrylates/C10-30 Alkyl Acrylate Crosspolymer erhältlich beispielsweise als Carbopol (Noveon). Die Phasen A und B wurden separat unter Rühren bei etwa 70°C hergestellt und dann unter weiterem Rühren miteinander vermischt. Nach Abkühlen auf etwa 50°C wurde die Phase C hinzugegeben. Nach weiterem Abkühlen wurde bei etwa 40°C die Phase D unter Rühren zugegeben und anschließend wurde homogenisiert.

### Beispiel 2A Sonnenschutzzubereitung II SPF 15

Die Phasen gemäß Beispiel I wurden ergänzt durch 0,5 % Komplex RPF-1, bestehend aus Wasser, 1 % Gelbildner, 7,5 % Phospholipide, 2 % Quebracho-Extrakt und 1 % Seidenraupen-Extrakt (gemäß WO 99/66881, Anspruch 1), bezogen auf das Gesamtgewicht des Komplexes, und weiterhin ergänzt durch 0,5 % 1,2-Hexanediol & Caprylyl Glycol & Tropolone.

### Beispiel 2B Sonnenschutzzubereitung II SPF 15

Die Phase D gemäß Beispiel I wurde ergänzt durch 0,5 % RPF-Komplex 1, bestehend aus Wasser q.s. ad 100%, 1 % Gelbildner, 7,5 % Phospholipide, 2 % Quebracho-Extrakt und 1 % Seidenraupen-Extrakt (gemäß WO 99/66881), bezogen auf das Gesamtgewicht des Komplexes. Der erfindungsgemäße RPF-Komplex 1 besteht somit aus den Bestandteilen a) bis e) der in der WO 99/66881 in Anspruch 1 beschriebenen kosmetischen Wirkstoffzubereitung mit hohem Radikalschutzfaktor in den oben genannten Gew.-%.

### Beispiel 2C Sonnenschutzzubereitung II SPF 15

In der Phase D gemäß Beispiel I wurden die 0,3 % Konservierungsmittel durch 0,5 % RPF-Komplex classic ersetzt, bestehend aus 3,5 % Lecithin, 2 % Angelica archangelica root extract, 2 % Pongamia pinnata seed extract, 2 % Camelia sinensis Leaf Extract, 2 % Coffea arabica seed extract, 8 % Glycerin, 8,25 % Alkohol denaturiert, 0,3 % Zitronensäure, 0,2 % Ascorbyl Palmitate, 0,2 % Tocopherol, 0,2 % Ascorbinsäure, 1,0 % Guar Hydroxypropyltrimonium chlorid, 0,2 % PEG-8, 0,28 % Konservierungsmittel und q. s. ad 100 Wasser, wobei der RPF-Komplex classic in Lecithin verkapselt vorliegt.

### Beispiel 3 Serumzubereitung I

| **Phase A** | |
|---|---|
| Wasser | q.s. ad 100 |
| Glycerine | 2,0 |
| Acrylates/C10-30 Alkyl Acrylate Crosspolymer | 0,2 |
| Allantoine | 0,05 |

| **Phase B** | |
|---|---|
| Triethanolamine | 0,02 |

| **Phase C** | |
|---|---|
| Sodium Acryloyldimethyl Taurate Copolymer | |
| & Isohexadecane | 6,0 |
| Sorbitan Oleate | 0,6 |
| Shea Butter | 2,0 |
| Bisabolol | 0,3 |

| **Phase D** | |
|---|---|
| Anthemis Nobilis Flower Extract | 1,0 |
| Calendula Oil | 1,0 |
| Boerhavia Diffusa Root Extract & Butylene Glycol | 1,0 |
| Hesperetin Laurate & PEG-6 Isostearate | 1,0 |
| Konservierungsmittel | 0,3 |
| Aroma Vanille | 0,2 |

Die Phase A wurde unter Rühren bei etwa 50°C hergestellt. Phase B wurde bei 40°C hinzugegeben und Phase C bei etwa 35°C.

### Beispiel 4A Serumzubereitung II

Die Phasen gemäß Beispiel 3 wurden wie in Beispiel 2A ergänzt. Der Anteil des RPF-Komplexes 1 betrug 0,8 %.

### Beispiel 4B Serumzubereitung II

Die Phase D gemäß Beispiel 3 wurde wie in Beispiel 2B ergänzt. Der Anteil des RPF-Komplexes 1 betrug 0,8 %. Zusätzlich wurden 0,5 % 1,2-Hexandiol & Caprylyl Glycol & Tropolone der Phase D zugesetzt.

### Beispiel 4C Serumzubereitung II

Die Phase D gemäß Beispiel 3 wurde wie in Beispiel 2C ergänzt. Zusätzlich wurden 0,5 % 1,2-Hexandiol & Caprylyl Glycol & Tropolone der Phase D zugesetzt.

### Beispiel 5 Cremezubereitung I

| **Phase A** | |
|---|---|
| Wasser | q.s. ad 100 |
| Glycerine | 2,5 |
| Ammonium Acryloyldimethyl Taurate | 0,5 |
| Xanthan Gum | 0,5 |
| Allantoin | 0,05 |
| Bisabolol | 0,3 |

| **Phase B** | |
|---|---|
| Polyglyceryl-3 Diisostearate | 0,5 |
| Beheneth-10 | 3,5 |
| Dicaprylyl Carbonate &Tocopherol | 4,5 |
| Shea Butter | 4,0 |

| **Phase C** | |
|---|---|
| Anthemis Nobilis Flower Extract | 1,0 |
| Calendula Oil | 1,2 |
| Boerhavia Diffusa Root Extract & Butylene Glycol | 0,8 |
| Hesperetin Laurate & PEG-6 Isostearate | 0,5 |
| Konservierungsmittel | 0,3 |
| Booster | 0,5 |
| Aroma Vanille | 0,1 |

Die Herstellung von Phase A und B erfolgte separat bei etwa 75°C. Bei dieser Temperatur wurden beide Phasen unter Rühren zusammengegeben. Nach Abkühlen auf etwa 40°C wurde Phase C unter Rühren zugegeben und anschließend das Gesamtgemisch homogenisiert.

### Beispiel 6A Cremezubereitung II

Die Phasen gemäß Beispiel 5 wurden wie in Beispiel 2A ergänzt. Der Anteil des RPF-Komplexes 1 betrug 0,3 %.

### Beispiel 6B Cremezubereitung II

Die Phase D gemäß Beispiel 5 wurde wie in Beispiel 2B ergänzt. Der Anteil des RPF-Komplexes 1 betrug 0,3 %.

### Beispiel 6C Cremezubereitung II

Die Phase D gemäß Beispiel 5 wurde wie in Beispiel 2C ergänzt. Der Anteil des RPF-Komplexes classic betrug 0,3 %.

### Beispiel 7 Vergleichsversuch

In einem Vergleichsversuch wurden die folgenden Produkte eingesetzt.
- das Serum gemäß Beispiel 3
- die Sonnenschutzzubereitung gemäß Beispiel 1
- die Cremezubereitung gemäß Beispiel 5.

Als Vergleichsformeln wurden zubereitet
- das Serum gemäß Beispiel 3 ohne die Bestandteile Anthemia Nobilis Flower Extract
   und Calendula Oil, und mit 0,9 % Konservierungsmittel anstelle von 0,3 %
   (Beispiel 3a)
- die Sonnenschutzzubereitung gemäß Beispiel 1 ohne die Bestandteile Anthemia Nobilis Flower Extract und Calendula Oil, sowie mit 0,9 % Konservierungsmittel anstelle von 0,3 % und mit 10 % Siliconöl Dimethicone anstelle von 3,0 % (Beispiel 1 a)
- die Cremezubereitung gemäß Beispiel 5 ohne die Bestandteile Anthemia Nobilis Flower Extract und Calendula Oil, und mit 0,9 % Konservierungsmittel anstelle von 0,3 %
   (Beispiel 5a)

12 Probanden im Alter von 23-54 Jahren vom kaukasischen Hauttyp und mit sensitiver Haut wurden wie folgt behandelt:
1) Ein Hautflächen-Testfeld von 5 x 12 cm jeweils auf dem linken und auf dem rechten Unterarm (Oberseite) wurde mit einem Serum gemäß Beispiel 3 (linker Unterarm) und mit einem Serum gemäß Beispiel 3a (rechter Unterarm) eingerieben mit einer Auftragsmenge von etwa 2 mg/cm².
2) Nach 15 Minuten Einwirkzeit wurde auf den gleichen Flächen des linken Unterarms eine Sonnenschutzzubereitung gemäß Beispiel 1 und auf dem rechten Unterarm eine Sonnenschutzzubreitung gemäß Beispiel 1a mit 2 mg/cm² aufgetragen.
3) Nach 20 Minuten Einwirkzeit wurde die Haut des Testfeldes mittels UV-Lichtsimulator (Xenonlampe mit Schottfilter WG 320 und UG 11/1 unter den Bedingungen des COLIPA-Verfahrens bestrahlt mit Bestrahlungszeiten von 5, 10 und 15 Minuten für jeweils ein Drittel des Testfeldes unter Abdeckung der anderen beiden Drittel.
4) Das Serum gemäß Beispiel 3 und Beispiel 3a wurde nach 10 Stunden nochmals wie unter 1) aufgetragen.
5) Nach 10 Minuten Einwirkzeit wurde die Cremezubereitung gemäß Beispiel 5 auf das Testfeld des linken Unterarms und die Cremezubereitung gemäß Beispiel 5a) auf das Testfeld des rechten Unterarm aufgetragen mit etwa 2 mg/cm².
6) Eine visuelle Bewertung der Hautrötung erfolgte 24 Stunden nach der Bestrahlung gemäß Punkt 3). Die Ergebnisse sind in der folgenden Tabelle 1 aufgeführt.

**Tabelle 1**

| Bestrahlungszeit | Hautrötung bei ... Anzahl der Probanden | | | |
|---|---|---|---|---|
| | keine | geringe | merkliche | starke |
| 5 Minuten | | | | |
| - Produkte Bsp. 3, 1, 5 | 12 | 0 | 0 | 0 |
| - Produkte Bsp. 3a, 1a, 5a | 7 | 3 | 2 | 0 |
| 10 Minuten | | | | |
| - Produkte Bsp. 3, 1, 5 | 12 | 0 | 0 | 0 |
| - Produkte Bsp. 3a, 1a, 5a | 6 | 4 | 2 | 0 |
| 15 Minuten | | | | |
| - Produkte Bsp. 3, 1, 5 | 12 | 0 | 0 | 0 |
| - Produkte Bsp. 3a, 1a, 5a | 3 | 4 | 4 | 1 |

Die Ergebnisse zeigen deutlich die Wirksamkeit der erfindungsgemäßen Anordnung von Produkten und die Kombination von deren Wirkstoffen. Über die gesamte Bestrahlungszeit wurde ein vollständiger Schutz der sensitiven Haut mit den Produkten der Beispiele 3, 1 und 5 erreicht. Dagegen kann bei sensitiver Haut der Schutz mit den üblichen Wirkstoffen nicht vollständig gewährleistet werden, und es kommt zu leichten bis merklichen Hautrötungen. Diese Haurötungen können teilweise auch durch Hautirritationen verursacht oder mitverursacht worden sein.

## Patentansprüche

1. Kosmetikset zur Verwendung zum Sonnenschutz von sensitiver Haut, **dadurch gekennzeichnet, dass** es umfasst
(a) eine Sonnenschutzzubereitung für den Bereich SPF 8 bis SPF 20 mit einem Gemisch von UVA- und UVB-Filtern und einem Gehalt an Siliconöl(en), der im Bereich von 1,2 bis 3,5 Gew.-% liegt, wobei der Gehalt an Siliconöl(en) auf das Gesamtgewicht der Sonnenschutzzubereitung bezogen ist;
(b) eine Serumzubereitung ohne Siliconöl; und
(c) eine Cremezubereitung ohne Siliconöl;
wobei alle Zubereitungen (a), (b) und (c) jeweils ein Wirkstoffgemisch aus Anthemis Nobilis Flower Extract, Hesperetin Laurate und Boerhavia Root Extract umfassen, sowie Calendula officinalis flower extract und der Gehalt an Konservierungsmitteln in allen Zubereitungen jeweils im Bereich von 0,01 bis 0,35 Gew.-% liegt, wobei der Gehalt an Konservierungsmitteln auf das Gesamtgewicht der jeweiligen Zubereitung bezogen ist, bei einer Lagerstabilität von wenigstens 24 Monaten für alle Zubereitungen; und
wobei das Verhältnis der Anteile des Wirkstoffgemisches in der Serumzubereitung zu den Anteilen des Wirkstoffgemisches in der Sonnenschutzzubereitung und in der Cremezubereitung im Bereich von
1 : 0,3 - 0,8 : 0,3 - 0,8 liegt.

2. Kosmetikset nach Anspruch 1, **dadurch gekennzeichnet, dass** der Anteil des Wirkstoffgemisches in der jeweiligen Zubereitung im Bereich von 0,9 bis 4 Gew.-% liegt, bezogen auf das Gesamtgewicht der jeweiligen Zubereitung.

3. Kosmetikset nach Anspruch 1, **dadurch gekennzeichnet, dass** der Anteil von Hesperetin Laurate in der jeweiligen Zubereitung im Bereich von 0,3 bis 2,0 Gew.-% liegt, bezogen auf das Gesamtgewicht der jeweiligen Zubereitung.

4. Sonnenschutz-Kosmetikset nach Anspruch 1, **dadurch gekennzeichnet, dass** der Anteil von Boerhavia Root Extract in der jeweilihen Zubereitung im Bereich von 0,3 bis 2,2 Gew.-% liegt, bezogen auf das Gesamtgewicht der jeweiligen Zubereitung.

5. Kosmetikset nach Anspruch 1, **dadurch gekennzeichnet, dass** das Gemisch von UVA- und UVB-Filtern 30 - 40 Gew.-% UVA-Filter enthält.

6. Kosmetikset nach Anspruch 1, **dadurch gekennzeichnet, dass** die Sonnenschutzzubereitung, die Serumzubereitung und die Cremezubereitung jeweils einen Radikalfänger oder ein Gemisch von Radikalfängern enthalten, ausgewählt unter Vitaminen, Vitaminderivaten, Flavonen, Flavonoiden, Enzymen und einem als RPF-Komplex 1 bezeichneten Gemisch von Quebracho blanco-Extrakt, Seidenraupen-extrakt, Hydro-Gel, Phospholipiden und Wasser.

7. Kosmetikset nach Anspruch 1, **dadurch gekennzeichnet, dass** der Gehalt an Calendula officinals flower extract in der jeweiligen Zubereitung im Bereich von 0,1 bis 2 Gew.-%, vorzugsweise im Bereich von 0,5 bis 1,5 Gew.-%, noch bevorzugter bei 1,0 oder 1,2 Gew.-% liegt, alle Prozentangaben bezogen auf das Gesamtgewicht der jeweiligen Zubereitung.

8. Kosmetikset nach Anspruch 1, **dadurch gekennzeichnet, dass** die Sonnenschutzzubereitung, die Serumzubereitung und die Cremezubereitung jeweils nicht mehr als 0,3 Gew.-% Konservierungsmittel enthalten, bezogen auf das Gesamtgewicht der jeweiligen Zubereitung.

## Claims

1. A cosmetic set for use for protecting sensitive skin against the sun, **characterized in that** it comprises
(a) a sunscreen preparation for the range SPF 8 to SPF 20 having a mixture of UVA and UVB filters and containing silicon oil(s) in the range of 1.2 to 3.5 wt. %, wherein the content of silicon oil(s) is based on the total weight of the sunscreen preparation;
(b) a serum preparation without silicon oil; and
(c) a cream preparation without silicon oil;
wherein all of the preparations (a), (b) and (c) each comprise an active mixture of Anthemis nobilis flower extract, Hesperetin Laurate and Boerhavia root extract, as well as Calendula officinalis flower extract and the content of preservatives in all of the preparations is, in each case, within the range of 0.01 to 0.35 wt. %, wherein the content of preservatives is based on the total weight of the respective preparation, with a storage stability of at least 24 months for all of the preparations; and
wherein the ratio of the portions of the active mixture in the serum preparation to the portions of the active mixture in the sunscreen preparation and in the cream preparation is within the range of 1 : 0.3 - 0.8 : 0.3 - 0.8.

2. The cosmetic set according to Claim 1, **characterized in that** the portion of the active mixture in the respective preparation is within the range of 0.9 to 4 wt. %, based on the total weight of the respective preparation.

3. The cosmetic set according to Claim 1, **characterized in that** the portion of Hesperetin Laurate in the respective preparation is within the range of 0.3 to 2.0 wt. %, based on the total weight of the respective preparation.

4. The sunscreen cosmetic set according to Claim 1, **characterized in that** the portion of Boerhavia root extract in the respective preparation is within the range of 0.3 to 2.2 wt. %, based on the total weight of the respective preparation.

5. The cosmetic set according to Claim 1, **characterized in that** the mixture of UVA and UVB filters contains 30 - 40 wt. % UVA filters.

6. The cosmetic set according to Claim 1, **characterized in that** the sunscreen preparation, the serum preparation and the cream preparation each contain a radical scavenger or a mixture of radical scavengers, selected from vitamins, vitamin derivatives, flavones, flavonoids, enzymes and a mixture of quebracho-blanco extract, silkworm extract, hydrogel, phospholipids and water designated RPF complex 1.

7. The cosmetic set according to Claim 1, **characterized in that** the content of Calendula officinalis flower extract in the respective preparation is within the range of 0.1 to 2 wt. %, preferably within the range of 0.5 to 1.5 wt. %, even more preferably 1.0 or 1.2 wt. %, all percentages being based on the total weight of the respective preparation.

8. The cosmetic set according to Claim 1, **characterized in that** the sunscreen preparation, the serum preparation and the cream preparation each contain no more than 0.3 wt. % preservatives, based on the total weight of the respective preparation.

## Revendications

1. Ensemble cosmétique utilisable pour la protection d'une peau sensible contre le rayonnement solaire, **caractérisé en ce qu'**il comprend
(a) une préparation de protection solaire pour la plage d'indices SPF 8 à SPF 20 avec un mélange de filtres UVA et UVB et une teneur en huile(s) de silicone comprise entre 1,2 et 3,5 % en poids, la teneur en huile(s) de silicone se rapportant au poids total de la préparation de protection solaire ;
(b) une préparation de sérum sans huile de silicone ; et
(c) une préparation de crème sans huile de silicone ;
chacune des préparations (a), (b) et (c) comprenant un mélange d'agents actifs à base d'extrait de fleurs d'Anthemis Nobilis, d'extraits de racine d'Hesperetin Laurate et de Boerhavia, et d'extrait de fleurs de Calendula officinalis et la teneur en conservateurs dans chacune des préparations étant comprise entre 0,01 et 0,35 % en poids, la teneur en conservateurs se rapportant au poids total de chaque préparation, pour une stabilité de stockage d'au moins 24 pour toutes les préparations ; et
le rapport entre les parts de mélange d'agents actifs dans la préparation de sérum et les parts de mélange d'agents actifs dans la préparation de protection solaire et dans la préparation de crème étant compris entre 1 : 0,3 et 0,8 : 0,3 - 0,8.

2. Ensemble cosmétique selon la revendication 1, **caractérisé en ce que** la part de mélange d'agents actifs dans chaque préparation est comprise entre 0,9 et 4 % en poids par rapport au poids total la préparation.

3. Ensemble cosmétique selon la revendication 1, **caractérisé en ce que** la part d'Hesperetin Laurate dans chaque préparation est comprise entre 0,3 et 2,0 % en poids par rapport au poids total la préparation.

4. Ensemble cosmétique de protection solaire selon la revendication 1, **caractérisé en ce que** la part d'extrait de racine de Boerhavia dans chaque préparation est comprise entre 0,3 et 2,2 % en poids par rapport au poids total de la préparation.

5. Ensemble cosmétique selon la revendication 1, **caractérisé en ce que** le mélange de filtres UVA et UVB contient entre 30 et 40 % en poids de filtres UVA.

6. Ensemble cosmétique selon la revendication 1, **caractérisé en ce que** la préparation de protection solaire, la préparation de sérum et la préparation de crème contiennent chacune un fixateur de radicaux ou un mélange de fixateurs de radicaux, sélectionné parmi des vitamines, des dérivés de vitamines, des flavones, des flavonoïdes, des enzymes et un mélange d'extrait de Quebracho blanco, d'extrait de ver à soie, d'hydrogel, de phospholipides et d'eau, qualifié de complexe RPF 1.

7. Ensemble cosmétique selon la revendication 1, **caractérisé en ce que** la teneur en extrait de fleurs de Calendula officinalis dans chaque préparation est comprise entre 0,1 et 2 % en poids, avantageusement entre 0,5 et 1,5 % en poids, préférentiellement sensiblement égale à 1,0 ou 1,2 % en poids, toutes les indications de pourcentage se rapportant au poids total de la préparation.

8. Ensemble cosmétique selon la revendication 1, **caractérisé en ce que** la préparation de protection solaire, la préparation de sérum et la préparation de crème ne contiennent chacune pas plus de 0,3 % en poids de conservateurs par rapport au poids total de la préparation.
